# FASCICULE DE BREVET EUROPEEN

(11) **EP 0 747 044 B1**
(45) Date de publication et mention de la délivrance du brevet: **18.04.2001**
(21) Numéro de dépôt: 96460018.3
(22) Date de dépôt: 06.05.1996
(51) Int. Cl.: A61K 7/48, A61K 35/08

(54) **Procédé de préparation d'eau de mer pour la désagrégation du cérumen**
Verfahren zur Herstellung von Meerwasser zur Auflösung von Cerumen
Process for production of seawater used for disintegrating cerumen

(30) Priorité: 08.06.1995 FR 9507030
(43) Date de publication de la demande: 11.12.1996
(73) Titulaire: Maugendre, Renée-Jane, 35400 Saint-Malo (FR)
(72) Inventeur: Maugendre, Renée-Jane, 35400 Saint-Malo (FR)
(74) Mandataire: Boulinguiez, Didier

(56) Documents cités:
- FR-A- 2 176 627
- FR-A- 2 242 971
- FR-A- 2 299 041
- FR-A- 2 546 754
- FR-A- 2 596 645
- FR-A- 2 688 133

## Description

La présente invention concerne un procédé de préparation d'eau de mer pour la désagrégation du cérumen. Un tel procédé peut notamment s'appliquer au cerumen d'origine humaine, mais il peut également être utilisé pour désagréger des bouchons de cérumen d'origine animale, comme par exemple de lapins, de chiens, de chats, etc.

L'accumulation de cérumen à l'intérieur du conduit auditif externe de l'oreille, qui se manifeste par la formation d'un "bouchon", pose de sérieux problèmes d'audition et nécessite toujours l'intervention d'un praticien. Ce dernier doit procéder au lavement du conduit auditif à l'aide d'un agent liquide approprié, le plus souvent de l'eau tiède. Une telle injection s'avère parfois insuffisante, pour désagréger de gros "bouchons".

On connaît par ailleurs des appareils d'hygiène de l'organe auditif à usage courant permettant le nettoyage du conduit externe de l'oreille. Ces appareils, tels que celui décrit dans le brevet FR-A-2 596 645, comprennent une pompe aspirante entraînée par un moteur électrique, ledit moteur étant alimenté par un variateur de vitesse, ainsi qu'un filtre à cérumen en tôle perforée visant à séparer les débris solides de cérumen de l'air aspiré.

Un inconvénient majeur de ce type d'appareil réside dans la nécessité de démonter périodiquement l'appareil pour nettoyer le filtre, en cas d'usage courant. Un autre inconvénient de cet appareil est que la séparation du cérumen du conduit auditif n'est pas systématiquement réalisée par le courant d'air, du fait de la forte adhérence du cérumen aux parois internes du conduit auditif. De plus, les vibrations engendrées par le moteur électrique sont susceptibles d'endommager le conduit auditif après un usage prolongé.

L'utilisation d' eau de mer diluée pour le lavage des fosses nasales a été décrite dans le brevet FR-A-2 299 041. Par ailleurs, l' utilisation d' eau de mer enrichie en un ou plusieurs oligo-éléments, pour le traitement et l' hygiène des voies respiratoires buccales, de la peau et des muqueuses gynécologiques a été décrite dans le brevet FR-A-2 688 133.

Un but de l'invention est de proposer un procédé de préparation d'eau de mer pour la désagrégation du cérumen, qui soit tel que la préparation obtenue puisse lyser tout "bouchon" de cérumen ou empêcher sa formation, quelle que soit sa taille et son origine.

A cet effet, un procédé selon l'invention de préparation d'eau de mer pour la désagrégation du cérumen consiste successivement en une séparation des particules solides d'un échantillon d'eau de mer et en une stérilisation dudit échantillon.

La préparation directement obtenue par ce procédé est d'un prix de revient raisonnable et elle désagrège efficacement le cérumen.

Avantageusement, ce procédé consiste à prévoir au moins deux étapes de pré-filtration dudit échantillon pour ladite séparation des particules solides.

L'eau de mer prélevée est ainsi débarrassée de ses impuretés.

Préférentiellement, le procédé selon l'invention consiste à prévoir au moins deux étapes de filtration pour ladite stérilisation de l'échantillon.

Selon une autre caractéristique de l'invention, la concentration massique des sels minéraux dans l'échantillon d'eau de mer est d'au moins 36 g/l.

Cette salinité minimale confère à la préparation finalement obtenue son bon pouvoir lytique vis-à-vis du cérumen.

Préférentiellement, lesdites particules solides séparées par pré-filtration sont d'une dimension supérieure à 1 µm.

Avantageusement, ledit échantillon stérilisé contient des sels minéraux et des oligo-éléments et les particules filtrées lors de la stérilisation sont d'une dimension inférieure ou égale à 0,22 µm.

Selon une autre caractéristique de l'invention, le procédé consiste à prévoir un transport de nuit de l'échantillon d'eau de mer pré-filtrée et un conditionnement adéquat pour ledit échantillon.

Avantageusement, ledit échantillon stérilisé est mis en contact avec de l'azote avant utilisation.

Ce système incluant un gaz inerte permet de réchauffer légèrement l'échantillon au contact du flacon pour lui conférer une activité lytique plus satisfaisante et un bon confort d'utilisation.

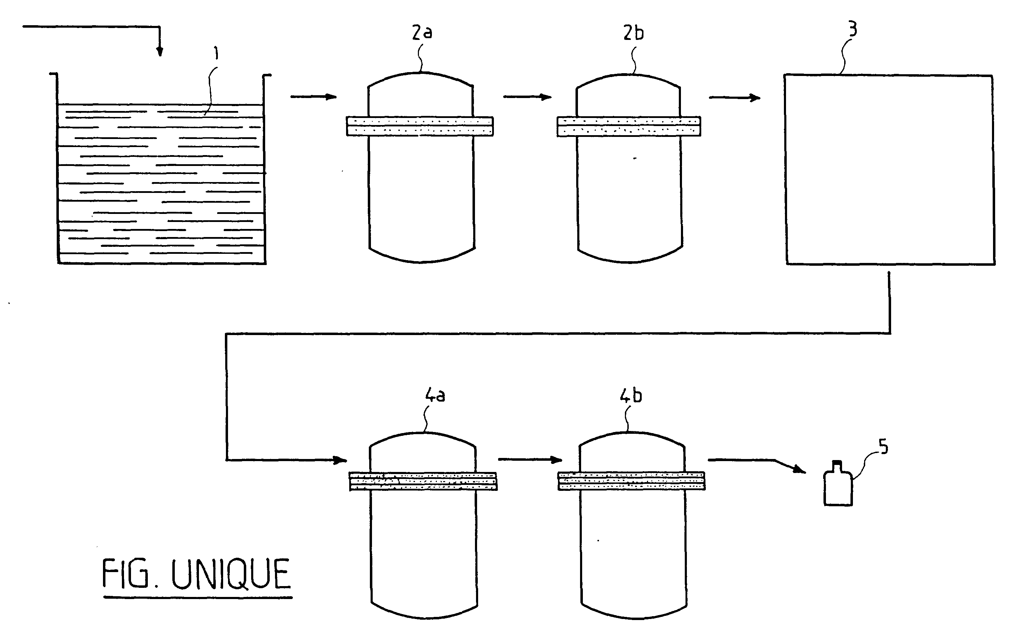

Les caractéristiques de l'invention mentionnée ci-dessus, ainsi que d'autres, apparaîtront plus clairement à la lecture de la description suivante d'un exemple de réalisation, ladite description étant faite en relation avec le dessin joint:

la Fig. unique est un schéma simplifié du procédé selon l'invention.

La première étape du procédé selon l'invention représenté sur cette Fig. unique consiste en un pompage d'échantillons 1 d'eau de mer naturelle, dont la concentration en sels minéraux est au moins égale à 36 g/l.

On fait ensuite subir à ces échantillons 1 d'eau de mer au moins deux pré-filtrations successives 2a, 2b visant à les débarrasser de leurs particules solides en suspension, les diamètres des mailles ou des pores utilisés étant respectivement de 5 µm et 1 µm. Les modes de filtration ultérieurement décrits sont donnés à titre indicatif et non limitatif.

La première opération de pré-filtration 2a peut consister par exemple en une filtration sur support à mailles de 5 µm, telle qu'un microtamisage sous pression. La seconde opération de pré-filtration 2b peut quant à elle consister en une filtration sur support épais. Dans ce type de microfiltration, le support permet non seulement une rétention en surface, mais également sur une certaine profondeur qui n'est cependant jamais importante. Les pores du support ont un diamètre égal à 1 µm, et ce support est tel que le liquide filtré rencontre une porosité décroissante dans le sens de la filtration.

Les échantillons d'eau de mer pré-filtrés et conditionnés dans des poches, puis dans des containers 3 de capacité avoisinant 1000 l, sont ensuite avantageusement transportés de nuit vers les unités de stérilisation avant conditionnement, les changements brusques de température étant ainsi évités. On cherche en effet à disposer d'une eau de mer dont la température est comprise entre 5 °C et 15 °C, ceci afin de lui conserver toutes ses propriétés cérumenolytiques pendant la durée du transport.

Conformément au procédé selon l'invention, les échantillons d'eau de mer pré-filtrés sont soumis à une stérilisation, qui consiste préférentiellement en au moins deux filtrations complémentaires 4a, 4b, dont les diamètres des pores sont respectivement de 0,5 µm et 0,22 µm. Ces filtrations 4a, 4b très fines peuvent par exemple s'effectuer sur support épais. Il est à noter que cette stérilisation permet de conserver tous les sels minéraux et oligo-éléments dans les échantillons d'eau de mer filtrés, ces sels minéraux jouant un rôle essentiel dans la désagrégation ultérieure du cérumen.

Comme on peut le voir sur la Fig. unique, les échantillons stérilisés d'eau de mer sont conditionnés dans des flacons 5 constitués d'un boîtier en aluminium. Avantageusement, ledit échantillon stérilisé est mis en contact avec un gaz inerte avant utilisation. Ce système vise à améliorer le contact entre l'eau de mer et le boîtier et permet ainsi de réchauffer de quelques degrés l'échantillon pour lui conférer une activité lytique plus satisfaisante ainsi qu'un bon confort d'utilisation. Le gaz choisi sera préférentiellement de l'azote, et la température idéale d'utilisation de l'échantillon sera comprise entre 25 et 30 °C.

La préparation d'eau de mer obtenue par le procédé selon l'invention peut être utilisée dans un dispositif de pulvérisation classique. Un tel dispositif est usuellement constitué d'un récipient destiné à recevoir l'eau de mer du flacon 5, d'une valve et d'un conduit dont une extrémité plonge dans l'eau de mer, l'autre étant reliée aux canaux d'amenée de ladite eau au niveau de l'embout de pulvérisation. Ledit conduit est parcouru par un gaz inerte sous pression qui propulse l'eau de mer à destination de l'embout de pulvérisation.

Il est à noter que cette préparation n'engendre aucune lésion du conduit auditif externe, comme l'ont montré des études de tolérance locale effectuées sur des lapins du type albinos. En effet, l'indice d'irritation primaire cutanée est nul pour ces lapins, ce qui signifie que le soluté obtenu par le procédé selon l'invention n'est absolument pas irritant pour la peau.

Par ailleurs, ledit soluté a fait l'objet d'études in vitro visant à évaluer précisément son effet lytique à l'égard de bouchons de cérumen prélevés chez l'homme. Le matériel utilisé consiste en des bouchons de cérumen humain de provenances diverses. On dispose également d'un bain marie et d'une étuve réglés respectivement à 35 °C et 70 °C.

Pour chaque essai, 5 ml d'un échantillon de bouchon de cérumen sont disposés dans un tube à essai en verre de 20 ml, du type commercialisé sous la marque déposée pyrex. Le tube à essai est placé au bain marie pendant 60 min. En fin d'essai, la partie liquide contenant les particules de bouchon en suspension est transvasée dans un tube sec puis déposée dans un verre de montre. Elle est ensuite mise à l'étuve pendant 24 heures et la masse de résidu, qui corespond à la fraction de cérumen désagrégée, est évaluée. La fraction restante, non attaquée, est également recueillie et mise à l'étuve. De plus, l'aspect et l'état des bouchons de cérumen sont observés 1min, 30 min et 60 min après la mise en contact desdits bouchons avec le soluté obtenu par le procédé selon l'invention. Cinq essais ont été réalisés sur des bouchons de cérumen de dimensions ou d'origine différente.

| **Essai N°1**: bouchon noir, compact, cassant de 90 mg. | | | |
|---|---|---|---|
| | T + 1 min | T + 30 min | T + 60 min |
| Echantillon 1 | désagrégation immédiate +++ | bouchon éclaté +++ particules surnageantes +++ | bouchon désagrégé +++ surnageant ++ |

Le poids de matière désagrégée recueillie est reporté ci-après. La quantité de sel retenue après évaporation a été soustraite (173 mg).

| | Poids en mg de matière désagrégée | % de désagrégation (ou lyse) |
|---|---|---|
| Echantillon 1 | 235 mg (- 173 mg) = 62 mg | 69 % |

| **Essai N°2:** bouchon couleur ambrée, cireux, compact de 72 mg. | | | |
|---|---|---|---|
| | T+1min | T+30min | T+60min |
| Echantillon 1 | 0 | bouchon | bouchon |
| | désagrégation | éclaté ++ | éclaté +++ |
| | | surnageant + | surnageant + |

Le poids de matière désagrégée est reporté ci-après.

| | Poids en mg de matière désagrégée | % de désagrégation (ou lyse) |
|---|---|---|
| Echantillon 1 | 203 mg (- 173 mg) = 30 mg | 42 % |

| **Essai N°3**: bouchon couleur ambrée, cireux compact de 224 mg. | | | |
|---|---|---|---|
| | T + 1 min | T + 30 min | T + 60 min |
| Bouchon 1 | début désagrégation pellicule blanche recouvre le bouchon | début désagrégation + | désagrégation + particules surnageant |

Le poids de matière désagrégée est reporte ci-apres. Dans cet essai, seule la fraction de bouchon non attaquée par le soluté a été pesée après dessication.

| | Poids en mg de matière désagrégée | % de désagrégation |
|---|---|---|
| Bouchon 1 | 224 mg (- 166 mg) = 58 mg | 26 % |

| **Essais N°4 et 5:** bouchon reconstitué à partir de 5 bouchons de provenances différentes. Ce bouchon est partagé en deux échantillons 1 et 3. | | | | |
|---|---|---|---|---|
| Echant. | Poids (mg) | T + 1 min | T + 30 min | T + 60 min |
| 1 | 190 | début | désagrégation +++ | désagrégation +++ |
| 3 | 159 | désagrégation ++ | surnageant ++ | surnageant ++ |

Le poids de matière désagrégée est calculé par différence après détermination du poids sec de la portion de bouchon non attaquée par les produits étudiés.

| | Poids en mg de matière désagrégée | % de désagrégation |
|---|---|---|
| Bouchon 1 | 190 mg (- 81) = 109 mg | 57 % |
| Bouchon 3 | 159 mg (- 53) = 106 mg | 67 % |

En conclusion, on obtient un fractionnement rapide et une désagrégation satisfaisante du bouchon de cérumen.

## Revendications

1. Procédé de préparation d'eau de mer pour la désagrégation du cérumen, caractérisé en ce qu'il consiste successivement en une séparation des particules solides d'un échantillon 1 d'eau de mer et en une stérilisation.

2. Procédé selon la revendication 1, caractérisé en ce qu'il consiste à prévoir au moins deux étapes de pré-filtration (2a, 2b) dudit échantillon 1 pour ladite séparation des particules solides.

3. Procédé selon la revendication 1, caractérisé en ce qu'il consiste à prévoir au moins deux étapes de filtration (4a, 4b) pour ladite stérilisation.

4. Procédé selon l'une des revendications précédentes, caractérisé en ce que la concentration massique des sels minéraux dans l'échantillon 1 d'eau de mer est d'au moins 36 g/l.

5. Procédé selon la revendication 2, caractérisé en ce que lesdites particules solides séparées sont d'une dimension supérieure à 1 µm.

6. Procédé selon l'une des revendications 1 ou 3, caractérisé en ce que ledit échantillon stérilisé contient des oligo-éléments et des sels minéraux.

7. Procédé selon la revendication 3, caractérisé en ce que les particules filtrées sont d'une dimension inférieure ou égale à 0,22 µm.

8. Procédé selon la revendication 2, caractérisé en ce qu'il consiste à prévoir un transport de nuit de l'échantillon d'eau de mer pré-filtrée et à une température variant entre 5 et 15°C.

9. Procédé selon la revendication 1, caractérisé en ce que ledit échantillon stérilisé est mis en contact avec de l'azote avant utilisation.

10. Utilisation d'eau de mer filtrée et stérilisée pour la réalisation d'une préparation destinée à prévenir et à supprimer les bouchons de cérumen d'origine humaine ou animale.

## Patentansprüche

1. Verfahren zur Aufbereitung von Meerwasser für die Desaggregation des Zerumens, dadurch gekennzeichnet, daß zunächst eine Abtrennung der festen Partikel aus einer Meerwasserprobe (1) und daran anschließend eine Sterilisation der Meerwasserprobe (1) durchgeführt wird.

2. Verfahren nach Anspruch 1, dadurch gekennzeichnet, daß mindestens zwei Vorfiltrationsschritten (2a, 2b) zur Durchführung der obigen Abtrennung der festen Partikel aus der Meerwasserprobe (1) vorgesehen sind.

3. Verfahren nach Anspruch 1, dadurch gekennzeichnet, daß mindestens zwei Filtrationsschritte (4a, 4b) zur Durchführung der obigen Sterilisation vorgesehen sind.

4. Verfahren nach einem der vorhergehenden Ansprüche, dadurch gekennzeichnet, daß die massebezogene Konzentration der anorganischen Salze in der Meerwasserprobe (1) mindestens 36 g/l beträgt.

5. Verfahren nach Anspruch 2, dadurch gekennzeichnet, daß die abgetrennten festen Partikel eine Größe von mehr als 1 µm aufweisen.

6. Verfahren nach Anspruch 1 oder 3, dadurch gekennzeichnet, daß die sterilisierte Meerwasserprobe (1) Spurenelemente und anorganische Salze enthält.

7. Verfahren nach Anspruch 3, dadurch gekennzeichnet, daß die filtrierten Partikel eine Größe von höchstens 0,22 µm aufweisen.

8. Verfahren nach Anspruch 2, dadurch gekennzeichnet, daß die vorfiltrierte Meerwasserprobe (1) in der Nacht und bei einer Temperatur im Bereich von 5 bis 15 °C transportiert wird.

9. Verfahren nach Anspruch 1, dadurch gekennzeichnet, daß die sterilisierte Probe vor ihrer Verwendung mit Stickstoff in Kontakt gebracht wird.

10. Verwendung von filtriertem und sterilisisertem Meerwasser für die Herstellung einer Zubereitung, die dafür vorgesehen ist, der Bildung von Zerumenpfropfen beim Menschen oder beim Tier vorzubeugen und/oder diese zu unterdrücken.

## Claims

1. A method of preparing sea water for the removal of cerumen, characterised in that it consists successively in separating solid particles from a sample 1 of sea water followed by sterilisation.

2. Method as claimed in claim 1, characterised in that it consists in incorporating at least two steps of pre-filtering (2a, 2b) said sample 1 for said separation of solid particles.

3. Method as claimed in claim 1, characterised in that it consists in incorporating at least two filtering steps (4a, 4b) for said sterilisation.

4. Method as claimed in one of the preceding claims, characterised in that the concentration by volume of mineral salts in the sample 1 of sea water is at least 36 g/l.

5. Method as claimed in claim 2, characterised in that said separated solid particles are of a dimension in excess of 1 µm.

6. Method as claimed in one of claims 1 or 3, characterised in that said sterilised sample contains trace elements and mineral salts.

7. Method as claimed in claim 3, characterised in that the filtered particles are of a dimension of less than or equal to 0.22 µm.

8. Method as claimed in claim 2, characterised in that it consists in transporting the sample of prefiltered sea water overnight and at a temperature ranging between 5 and 15°C.

9. Method as claimed in claim 1, characterised in that said sterilised sample is placed in contact with nitrogen before use.

10. Use of filtered and sterilised sea water in making a preparation intended to prevent and remove earwax plugs of human or animal origin.
